# EUROPEAN PATENT SPECIFICATION

(11) **EP 0 370 733 B1**
(45) Date of publication and mention of the grant of the patent: **11.10.1995**
(21) Application number: 89312013.9
(22) Date of filing: 20.11.1989
(51) Int. Cl.: A61B 17/34, A61M 1/00

(54) **Bendable trocar**
Biegbarer Trokar
Trocart pliable

(30) Priority: 21.11.1988 US 273547
(43) Date of publication of application: 30.05.1990
(73) Proprietor: JOHNSON & JOHNSON ORTHOPAEDICS INC., New Brunswick New Jersey 08903 (US)
(72) Inventor: Broadnax, Cecil Harridell, Jr., Somerset, NJ 08873 (US)
(74) Representative: Fisher, Adrian John

(56) References cited:
- EP-A- 0 059 620
- DE-U- 8 712 927
- FR-A- 2 161 950
- FR-A- 2 512 677
- GB-A- 1 002 505

## Description

### FIELD OF THE INVENTION

The present invention relates to trocars or needles which are used to thread or insert wound drainage catheters into the body of a surgical patient. The trocar of the present invention can be bent to the desired curvature prior to use.

### BACKGROUND OF THE INVENTION

Wound drain catheters generally have a flexible drain portion and a flexible outflow tube portion. The drain portion is integral or attached to the outflow portion. The drain portion is placed in or adjacent the wound site and the outflow portion will pass through the skin of the patient and be connected to a source of vacuum to drain the wound site.

Trocars are commonly used to insert wound drainage catheters or tubing into a drainage site adjacent a surgical wound or from a surgical wound site through the skin of a patient. These trocars usually have the catheter or tubing attached to the one end of the trocar so that the tubing follows the trocar along a path through the patient's body.

Several techniques may be used to insert a wound drain catheter in the patient's body. For example, a surgeon may simply place the drain portion and a small part of the outflow tube portion in the wound, close the incision, and suture around the outflow tube portion. This technique is somewhat unsatisfactory, since it is difficult to completely seal the area around the outflow tube by suturing, and thus, the wound may become infected. A more satisfactory technique is to pass a trocar, preattached to the end of the outflow tube, through healthy tissue by entering the patient's body at a point within the wound and exiting at a point adjacent to the wound. The surgeon pulls the outflow tube portion through the tissue with the trocar until the catheter is properly positioned, with the drain in the wound. Since the outflow tube exits the body at a point adjacent the wound, the wound can be completely closed by suturing, thereby reducing the risk of infection.

The trocars are manufactured with a slight bend near the pointed end to allow them to be manipulated through the skin of the patient. The catheter or drainage tubing will follow the path made by the trocar until the drainage end of the tubing is in its desired position within the body and the outflow portion of the catheter outside the patient's body. At that time the trocar will then be cut from the tubing and the tubing connected to a wound drain evacuator or a source of vacuum.

U.S. Patent No. 4,398,910 discloses the use of trocar in the manner described above.

U.S. Patent No. 4,359,053 discloses the fastening of plastic tubing to a trocar or needle for the same purpose.

The trocars are usually made of very hard surgical grade stainless steel or other materials so that they may be sharpened to a very fine point to allow the distal end or sharpened end of the trocar to pass through the body tissue. There is generally a slight bend, about 15°, in the trocar when manufactured as shown in U.S. Patent No. 4,398,910 to allow the trocar to be manipulated through the body of the patient to correctly position the wound drainage tubing in the position desired by the surgeon. In many instances, it is difficult to properly position the wound drainage tubing because it is difficult to pass the trocar through the patient's body without hitting a solid structure such as bone.

DE-U-8712927 discloses a needle-like instrument for the subcutaneous introduction of catheters. The catheter may be attached to the trailing end of the instrument; the leading end of the instrument is furnished with a hemispherical head. The instrument is constructed from a soft stainless steel so as to be bendable.

A bendable trocar as defined in the pre-characterising part of claim 1 is known from FR-A-2 512 677.

### SUMMARY OF THE INVENTION

The present invention provides a trocar as defined in claim 1, normally used to insert wound drainage tubing into the body of a patient, which is capable of being readily bent by the surgeon to the configuration which is desired by the surgeon. This allows easier and more accurate insertion of the wound drain catheter in the desired location in the patient's body. The trocar has an area of reduced cross section which allows the trocar to be bent with a force which can be generated by the hands of the surgeon.

### BRIEF DESCRIPTION OF THE DRAWINGS

In the drawings,
Fig. 1 shows the trocar of the present invention with the point of the trocar shown in phantom in two different positions .
Fig. 2 shows the trocar of the present invention in a straight configuration.
Fig. 3 shows the trocar of the prior art.
Fig. 4A shows the utilization of the trocar of the present invention and the intended path of the trocar through a body.
Fig. 4B shows the position of the wound drain catheter after it has been inserted in the body following the path of the trocar.

### DETAILED DESCRIPTION OF THE INVENTION

The bendable trocar of the present invention comprises a generally circular shaft 10 which has a triangular point 11 at the distal end and a tubing attachment fixture 12 at the proximal end. At a location which is preferably between the midpoint of the trocar and the distal end there is a reduced cross section region 13 which is tapered at both ends 14. The trocar is made of a material which is capable of being sharpened to a fine point. The material is sufficiently hard to hold the point of the trocar. The trocar must also be capable of being sterilized before use. Although numerous materials would fit these criteria, such as malleable metals and including some hard plastic materials, the material of choice is a grade 303 stainless steel. The overall length of the trocar is commonly approximately six inches (15.2 cm). The center of the reduced cross section region 13 is between two and two and three quarter inches (.50 - 70 mm) from the distal end or pointed end of the trocar. This position can be varied, but generally, the reduced section should be no more than one-half of the distance between the distal end and the proximal end of the trocar. The reduction in the cross sectional area of the shaft of the trocar should be such that the trocar can be readily bent by the surgeon to the desired configuration. However, the trocar should not be so flexible that itself could bend upon hitting a hard object, such as a bone in the body when the trocar is in use. The trocar can be bent up to an angle of about 60° to 90° without breaking.

Trocar of the type used to insert wound drainage tubing or catheters have a diameter of from about 0.125 to .250 inches (3 to 6.5 mm) and a length of about 6 inches (150 mm). If the trocar has a diameter of about 0.125 inches (3 mm) or less, it can be bent without any reduction in its cross sectional area but it can be bent much more readily if the cross section is reduced. If the trocar has a diameter over about .150 inches (3.8 mm) it is difficult to bend. Reducing the cross section of the trocar to about 0.08 to 0.1 inches (2 to 2.5 mm) will allow the trocar to be bent to the desired configuration by the surgeon.

Generally, the amount of force desired to bend the trocar to the desired position is between 44.5 and 178 N (10 and 40 pounds), preferably 89 to 133.5 N (20 to 30 pounds). This amount of force can readily be exerted by a surgeon with his hands. The amount of reduction in the cross sectional area of the shaft is somewhere between 8% and 20%, but generally, with grade 303 stainless steel a reduced shaft diameter of approximately 3/32 of an inch, (2 to 3 millimeters), can readily be bent by the surgeon to the desired configuration. There is a taper 14 from the large diameter portion 15 to the reduced diameter portion 13 at each end of the reduced diameter portion. The taper 14 helps to prevent tissue from being caught in the reduced diameter section when the trocar is drawn through the tissue.

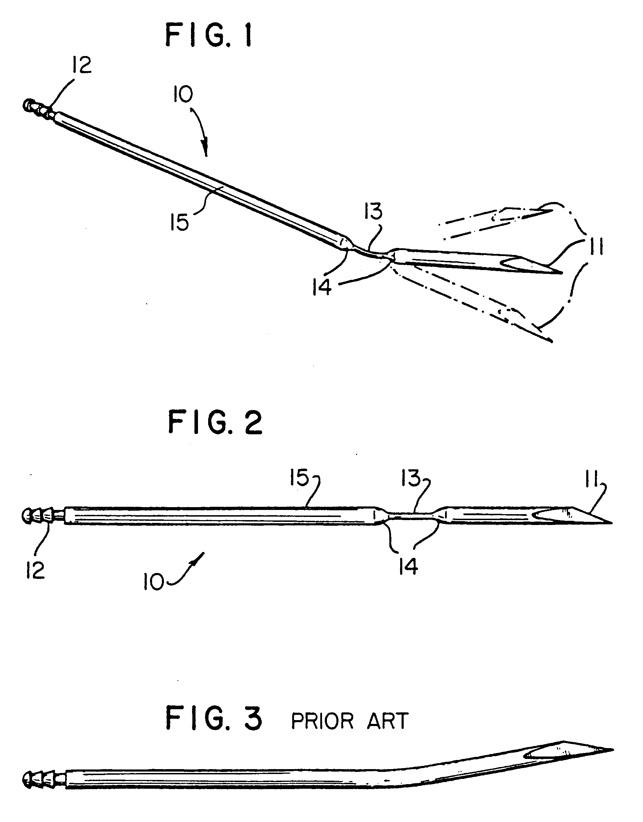

It should be noted that in the constant diameter of the prior art trocar shown in Fig. 3, is such that it is very difficult, if not impossible, to bend the shaft of the trocar to any great degree.

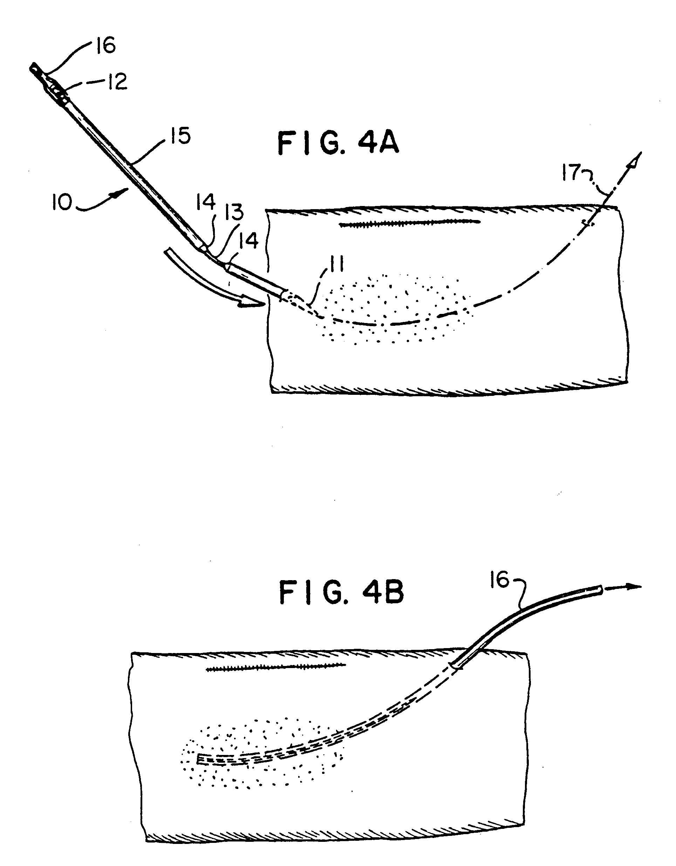

In use, as shown in Figs. 4A and 4B, the catheter 16 would be attached to the proximal end of the trocar. The trocar would be bent to the desired configuration and passed through the surgical flap in the skin of the patient following a curved track as shown in the dotted line 17. Normally the trocar would be inserted through the patient's skin from the operative site. The catheter 16 would follow in the path of the trocar, and after the trocar had been removed from the patient the tubing would be placed in the correct position to drain the wound of the patient as shown in Fig. 4B. The trocar would then be cut from the tubing and the tubing inserted into a drain evacuator as well-known in the art.

## Claims

1. A trocar (10) comprising a bendable shaft (15) having a cutting edge (115) formed from the material of the shaft at one end, and means (12) to attach a flexible tubing to the opposite end of the shaft (15), characterised in that said shaft (15) has a region (13) of reduced, circular cross section having a diameter less than the unreduced diameter of the shaft, to allow the shaft (15) to be bent by the user to an appropriate angle to allow the user to manipulate the trocar (10) through the skin of a patient, the remainder of the shaft being rigid.

2. The trocar (10) according to claim 1 in which the shaft (15) is of circular section.

3. The trocar (10) of claim 1 or claim 2 in which the region (13) of reduced cross section is at a location between the midpoint of the shaft (15) and its cutting edge (11).

4. The trocar (10) according to any preceding claim in which the shaft (15) is made of stainless steel.

5. The trocar (10) according to any preceding claim in which the region (13) of reduced cross section has a diameter between 8% and 20% of the unreduced diameter of the shaft.

6. The trocar (10) of any preceding claim in which there is a taper (14) at each end of the region (13) of reduced cross section of the shaft (15).

## Patentansprüche

1. Trokar (10) mit einem biegbaren Schaft (15) mit einem Schnittrand (115) aus dem Material des Schafts an einem Ende und mit einer Einrichtung (12) zum Anbringen eines flexiblen Schlauchs am anderen Ende des Schafts (15), dadurch **gekennzeichnet**, daß der Schaft (15) starr ist und einen Bereich (13) mit verringertem, kreisförmigen Querschnitt mit einem Durchmesser aufweist, der kleiner ist als der nicht verringerte Durchmesser des Schafts, damit der Schaft (15) vom Benutzer in einem geeigneten Winkel abgebogen werden kann, um es dem Benutzer zu ermöglichen, den Trokar (10) durch die Haut eines Patienten zu manipulieren.

2. Trokar (10) nach Anspruch 1, wobei der Schaft (15) einen kreisförmigen Querschnitt hat.

3. Trokar (10) nach Anspruch 1 oder Anspruch 2, wobei sich der Bereich (13) mit verringertem Querschnitt an einer Stelle zwischen der Mitte des Schaftes (15) und seinem Schnittrand (11) befindet.

4. Trokar (10) nach einem der vorstehenden Ansprüche, wobei der Schaft (15) aus rostfreiem Stahl ist.

5. Trokar (10) nach einem der vorstehenden Ansprüche, wobei der Bereich (13) mit verringertem Querschnitt einen Durchmesser zwischen 8% und 20% des nicht verringerten Durchmessers des Schaftes aufweist.

6. Trokar (10) nach einem der vorstehenden Ansprüche, mit einer konischen Verjüngung (14) an jedem Ende des Bereiches (13) des Schaftes (15) mit verringertem Querschnitt.

## Revendications

1. Trocart (10) comprenant une tige cintrable (15) comportant une arête tranchante (11) formée à partir du matériau de la tige au niveau d'une extrémité, et un moyen (12) pour fixer un tubage flexible à l'extrémité opposée de la tige (15), caractérisé en ce que ladite tige (15) est rigide et comporte une région (13) présentant une section en coupe circulaire réduite dont le diamètre est inférieur au diamètre non réduit de la tige, pour permettre à la tige (15) d'être cintrée par l'utilisateur selon un angle approprié pour permettre à l'utilisateur de manipuler le trocart (10) au travers de la peau d'un patient.

2. Trocart (10) selon la revendication 1, dans lequel la tige (15) présente une section circulaire.

3. Trocart (10) selon la revendication 1 ou 2, dans lequel la région (13) présentant une section en coupe réduite est en un emplacement entre le point médian de la tige (15) et son arête tranchante (11).

4. Trocart (10) selon l'une quelconque des revendications précédentes, dans lequel la tige (15) est réalisée en acier inoxydable.

5. Trocart (10) selon l'une quelconque des revendications précédentes, dans lequel la région (13) de section en coupe réduite présente un diamètre se situant entre 8 % et 20 % du diamètre non réduit de la tige.

6. Trocart (10) selon l'une quelconque des revendications précédentes, dans lequel il y a un cône (14) au niveau de chaque extrémité de la région (13) de section en coupe réduite de la tige (15).
